# EUROPÄISCHE PATENTSCHRIFT

(11) **EP 0 433 883 B1**
(45) Veröffentlichungstag und Bekanntmachung des Hinweises auf die Patenterteilung: **24.03.1993**
(21) Anmeldenummer: 90124061.4
(22) Anmeldetag: 13.12.1990
(51) Int. Cl.: C07C 255/29, C07C 253/30

(54) **Verfahren zur Herstellung von alpha-Formylaminonitrilen**
Process for the preparation of alpha-formylaminonitriles
Procédé pour la préparation d'alpha-formylaminonitriles

(30) Priorität: 22.12.1989 DE 3942575
(43) Veröffentlichungstag der Anmeldung: 26.06.1991
(73) Patentinhaber: BASF Aktiengesellschaft, 67063 Ludwigshafen (DE)
(72) Erfinder: Fikentscher, Rolf, Dr., W-6700 Ludwigshafen (DE); Kroener, Michael, Dr., W-6800 Mannheim 31 (DE)

(56) Entgegenhaltungen:
- EP-A- 0 205 131
- DE-A- 1 950 280
- CHEMICAL ABSTRACTS, Band 73, Nr. 7, 17. August 1970, Columbus, Ohio, USA; F. BECKE et al. "New synthesis of alpha(N-formylamino) nitriles, alpha-(N-formylamino)amides and of alpha- and beta-amino acids" Seite 297, Spalte 2, Zusammenfassung-Nr. 34 991w

## Beschreibung

Die vorliegende Erfindung betrifft ein Verfahren zur Herstellung von α-Formylaminonitrilen der allgemeinen Formel I 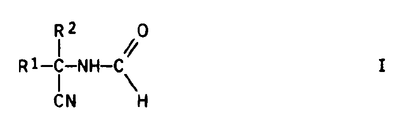
in der die Reste R¹ und R² für Wasserstoff oder für Wasserstoff und unsubstituierte oder mit unter den Reaktionsbedingungen inerten Substituenten substituierte, aliphatische oder heteroaliphatische Reste mit 1 bis 10, cycloaliphatische oder heterocycloaliphatische Reste mit 3 bis 6, araliphatische Reste mit 7 bis 12, heteroaraliphatische Reste mit 4 bis 12, aromatische Reste mit 6 bis 10 oder heteroaromatische Reste mit 3 bis 10 Kohlenstoffatomen stehen,
durch Umsetzung von Cyanhydrinen der allgemeinen Formel II 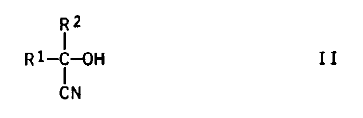
mit Formamid III 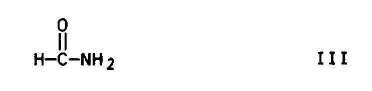
in Gegenwart von Säuren.

Gemäß der Lehre von DE-A 19 50 280 sind α-Formylaminonitrile der Formel I mit R¹ und/oder R² ungleich Wasserstoff durch die säurekatalysierte Umsetzung der entsprechenden Cyanhydrine II mit Formamid III bei Temperaturen von 60 bis 180°C erhältlich. Dabei wird bevorzugt mit einem Formamid-überschuß von 2 bis 3 Mol pro Mol Cyanhydrin II gearbeitet.

Das Verfahren der DE-A 19 50 280 liefert zwar gute Ergebnisse bei der Herstellung von α-Formylaminonitrilen, beispielsweise ist α-Formylalaninnitril IV 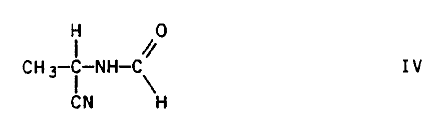
auf diese Weise in einer Ausbeute von 82 % ausgehend von Acetaldehydcyanhydrin erhältlich, dennoch erschien das Verfahren in einigen Punkten als verbesserungsfähig. So entstehen z.B. bei der Herstellung von α-Formylalaninnitril nach dem Verfahren dieser Schrift aufgrund einer Reihe nicht näher bekannter Nebenreaktionen pro Liter Reaktionsflüssigkeit 4 bis 6 l Abgas, das im wesentlichen aus einem Kohlenmonoxid/Kohlendioxid-Gasgemisch besteht, welches noch etwa 5 Vol.% Cyanwasserstoff enthält und daher unter kostenaufwendigen Sicherheitsmaßnahmen sorgfältig entsorgt werden muß. Infolge dieser Gasentwicklung und damit verbundener Siedeverzüge ist die sichere Reaktionsführung erschwert, weswegen der Reaktor mit zusätzlichen, der Betriebssicherheit dienenden Vorrichtungen ausgerüstet werden muß.

Infolge dieser Nebenreaktionen beträgt die Formylaminonitril-Ausbeute bei diesem Verfahren bezüglich des eingesetzten Formamids III nur ca. 75 % der Theorie. Des weiteren wird unter den angewandten Reaktionsbedingungen die Nitrilgruppe von I und II durch das Reaktionswasser partiell zum Carbonsäureamid hydrolysiert, wodurch gleichfalls Ausbeuteverluste entstehen.

Der vorliegenden Erfindung lag deshalb die Aufgabe zugrunde, Maßnahmen zu finden, welche es ermöglichen, die nachteiligen Begleiterscheinungen dieses Verfahrens zu beheben und seine Wirtschaftlichkeit und seine Ausbeute zu verbessern.

Dementsprechend wurde ein Verfahren zur Herstellung von α-Formylaminonitrilen der allgemeinen Formel I 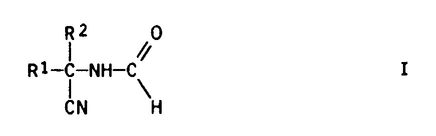
in der die Reste R¹ und R² für Wasserstoff oder für Wasserstoff und für unsubstituierte oder mit unter den Reaktionsbedingungen inerten Substituenten substituierte, aliphatische oder heterocycloaliphatische Reste mit 1 bis 10, cycloaliphatische oder heteroaliphatische Reste mit 3 bis 6, araliphatische Reste mit 7 bis 12, heteroaraliphatische Reste mit 4 bis 12, aromatische Reste mit 6 bis 10 oder heteroaromatische Reste mit 3 bis 10 Kohlenstoffatomen stehen,
durch Umsetzung von Cyanhydrinen der allgemeinen Formel II 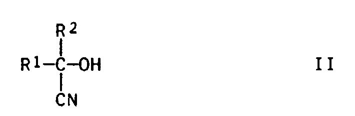
mit Formamid III 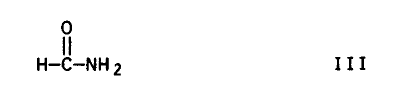
in Gegenwart von Säuren, gefunden, das dadurch gekennzeichnet ist, daß man der Reaktionsmischung mindestens ein Ammoniumsalz zusetzt.

Im erfindungsgemäßen Verfahren werden also Cyanhydrine II mit Formamid III gemäß Gleichung (1) 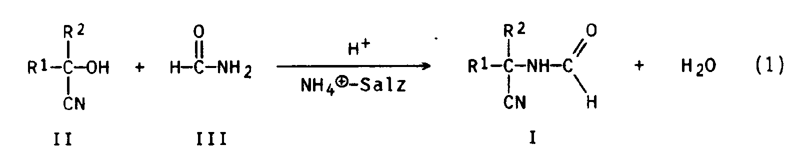
unter Säurekatalyse und unter Zusatz eines oder mehrerer als Cokatalysatoren wirkender Ammoniumsalze zu den entsprechenden α-Formylaminonitrilen I umgesetzt.

Zu diesem Zweck können erfindungsgemäß im Prinzip Ammoniumsalze mit beliebigen Säureanionen, wie Formiat, Acetat, Propionat, Halogenid, Hydrogensulfat, Dihydrogenphosphat, Methansulfonat, Toluolsulfonat, Lactat, Oxalat, Adipat, Citrat usw. verwendet werden. Bevorzugt werden im erfindungsgemäßen Verfahren Ammoniumsalze der Säuren verwendet, welche zur Säurekatalyse der erfindungsgemäßen Umsetzung dienen. Dabei sind die Ammoniumsalze der niederen Carbonsäuren, insbesondere der C₁- bis C₆-Mono-carbonsäuren bevorzugt. Besonders vorteilhaft ist der Gebrauch von Ammoniumformiat. Die Ammoniumsalze, insbesondere das Ammoniumformiat, werden der Reaktionsmischung zweckmäßigerweise in Mengen von 0,05 bis 0,5 mol, vorzugsweise in Mengen von 0,1 bis 0,3 mol pro Mol Cyanhydrin II zugesetzt. Höhere Ammoniumsalz-Zusätze sind möglich. Selbstverständlich können auch Gemische verschiedener Ammoniumsalze dem Reaktionsgemisch zugesetzt werden, im allgemeinen wird jedoch der Zusatz eines Ammoniumsalzes bevorzugt.

Zur Katalyse der erfindungsgemäßen Umsetzung können sowohl Mineralsäuren als auch organische Carbon- und/oder Sulfonsäuren verwendet werden. Vorzugsweise werden niedere aliphatische Mono-, Di- oder Tricarbonsäuren, insbesondere C₁- bis C₆-Monocarbonsäuren und besonders vorteilhaft, Ameisensäure verwendet. Die Verwendung substituierter Carbonsäuren, beispielsweise Hydroxycarbonsäuren oder halogenierter Carbonsäuren, wie Chloressigsäure und Trifluoressigsäure ist ebenfalls möglich. Die Säuren werden in der Regel in Mengen von 0,001 bis 1, vorzugsweise in Mengen von 0, 1 bis 0, 3 mol Säure pro Mol Cyanhydrin eingesetzt. Selbstverständlich können auch Gemische verschiedener Säuren Anwendung finden, vorzugsweise wird aber aus praktischen Gründen, beispielsweise wegen der einfacheren Aufarbeitung der Reaktionsmischung, nur eine Säurespezies als Katalysator benutzt.

Das erfindungsgemäße Verfahren kann mit bezüglich dem Cyanhydrin II äquimolaren Mengen an Formamid III durchgeführt werden, vorzugsweise werden dabei aber Formamidüberschüsse angewandt. In der Regel wird mit 1,1 bis 2,0 mol, vorzugsweise 1,2 bis 1,8 mol Formamid pro Mol Cyanhydrin II gearbeitet aber es sind auch höhere Überschüsse möglich.

Die erfindungsgemäße Umsetzung wird im allgemeinen bei Temperaturen von 20 bis 150°C, vorzugsweise von 60 bis 120°C und besonders vorteilhaft bei Temperaturen von 80 bis 100°C durchgeführt. Bei Temperaturen oberhalb 120°C ist die Umsetzung innerhalb weniger Minuten beendet, weshalb in diesem Temperaturbereich vorzugsweise mit kontinuierlich betriebenen Rohrreaktoren gearbeitet wird, bei denen sich eine derart kurze Verweilzeit der Reaktionsmischung einstellen läßt. Reaktionstemperaturen von 60 bis 120°C, insbesondere von 80 bis 100°C, können sowohl in kontinuierlich als auch in diskontinuierlich betriebenen Anlagen vorteilhaft angewandt werden. Bei Temperaturen unterhalb 60°C wird aufgrund der verlängerten Reaktionszeit zweckmäßigerweise im chargenweisen Betrieb gearbeitet. So erhält man beispielsweise durch einfaches Stehenlassen der in einem Behälter vermischten Reaktanten bei Raumtemperatur innerhalb weniger Tage das gewünschte α-Formylaminonitril in praktisch quantitativer Ausbeute bezüglich des eingesetzten Cyanhydrins. Eine solche Vorgehensweise kann beispielsweise in kleineren Betrieben oder für kleinere Chargen besonders wirtschaftlich sein.

Das erfindungsgemäße Verfahren wird im allgemeinen so durchgeführt, daß man die Reaktanten im Reaktor vorlegt, mischt und auf die vorgesehene Reaktionstemperatur erwärmt. Dabei wird die Umsetzung vorzugsweise unter dem Eigendruck des Reaktionssystems durchgeführt. Im chargenweisen Betrieb werden daher vorzugsweise Rührautoklaven, im kontinuierlichen Verfahren vorzugsweise druckstabile Rohrreaktoren oder besonders vorteilhaft, Rührkesselkaskaden verwendet. Der flüssigen Reaktionsmischung kann gewünschtenfalls noch ein Solvens zugesetzt werden, doch ist dies in der Regel nicht erforderlich.

Nach beendeter Umsetzung wird die Reaktionsmischung im allgemeinen destillativ aufgearbeitet, es ist aber auch möglich, das gewünschte Produkt durch Extraktion oder Kristallisation zu isolieren. Im Falle der destillativen Aufarbeitung wird das überschüssige Formamid und je nach verwendeter Katalysatorsäure, insbesondere bei Verwendung von Carbonsäuren, die Säure sowie, je nach seiner Destillierbarkeit, das α-Formylaminonitril I destillativ vom Ammoniumsalz, welches als Sumpfprodukt anfällt, abgetrennt. Das Produkt I wird zur Weiterverarbeitung isoliert, wohingegen die Katalysatorsäure, das Formamid und das Ammoniumsalz je nach Bedarf in die Umsetzung zurück- oder anderen Verwendungszwecken zugeführt werden können.

Das vorliegende Verfahren ist praktisch universell zur Herstellung von α-Formylaminonitrilen aus den entsprechenden Cyanhydrinen geeignet. So können die Cyanhydrine II, in denen R¹ und R² für Wasserstoff oder für Wasserstoff und für unsubstituierte oder mit unter den Reaktionsbedingungen inerten Substituenten substituierte, aliphatische oder heteroaliphatische Reste mit 1 bis 10, cycloaliphatische oder heterocycloaliphatische Reste mit 3 bis 6, araliphatische Reste mit 7 bis 12, heteroaraliphatische Reste mit 4 bis 12, aromatische Reste mit 6 bis 10 oder heteroaromatische Reste mit 3 bis 10 Kohlenstoffatomen stehen, im Verfahren verwendet werden.

Die heteroaliphatischen Reste R¹ oder R² können je nach ihrer Größe bis zu 5 Sauerstoffatome in Ether- oder Polyethergruppierungen enthalten. Des weiteren können die aliphatischen Reste R¹ und R² geradkettig, verzweigt oder miteinander zu einem 5- bis 6-gliedrigen Ring verbunden sein. Die Heterocycloalkyl-, Heteroaralkyl- und Heteroarylreste R¹ oder R² können im allgemeinen 1 oder 2 der Heteroatome Stickstoff, Sauerstoff und/oder Schwefel enthalten.

Um die Breite der Anwendbarkeit des erfindungsgemäßen Verfahrens zu zeigen, seien im folgenden eine Reihe von Aldehyden oder Ketonen beispielhaft aufgezählt, aus deren Cyanhydrinen II sich nach dem erfindungsgemäßen Verfahren vorteilhaft α-Formylaminonitrile produzieren lassen:
Formaldehyd, Acetaldehyd, Propionaldehyd, n-Butyraldehyd, Isobutyraldehyd, n-Valeraldehyd, Isovaleraldehyd, 2-Methyl-butyraldehyd, Hexanal, Benzaldehyd, Phenylacetaldehyd, Phenylpropionaldehyd, Phenylisobutyraldehyd, 4-Methylphenyl-acetaldehyd, 4-Methoxyphenyl-acetaldehyd, 4-Chlorphenyl-acetaldehyd, 2,6-Dichlorbenzaldehyd, 4-Hydroxiphenyl-acetaldehyd, 4-Chlorbenzaldehyd, Cyclopropancarbaldehyd, Cyclohexancarbaldehyd, 1-Methylcyclopropancarbaldehyd, 2-Pyrrolidon-3-propionaldehyd, Tetrahydropyran-3-aldehyd, 2-(1,4-Dioxan)-carbaldehyd, Tetrahydrothiopyran-3-carbaldehyd, Pyridin-2-carbaldehyd, Pyridin-3-carbaldehyd, Furan-3-carbaldehyd, Furfurol, Pyrrol-2-carbaldehyd, Imidazolyl-4-acetaldehyd, Thiophen-3-aldehyd, (1H)-Indolyl3-acetaldehyd.

Des weiteren können nach dem erfindungsgemäßen Verfahren α-N-Formylaminonitrile I hergestellt werden, deren heteroaliphatische Reste R¹ oder R² als Heteroatome vorzugsweise Sauerstoffatome und zwar in Ethergruppierungen enthalten. Die Anzahl der Sauerstoffatome pro heteroaliphatischem Rest kann dabei bis zu 5 betragen, wobei diese Anzahl selbstverständlich von der Anzahl der Kohlenstoffatome im heteroaliphatischen Rest abhängig ist. Zur Veranschaulichung seien beispielhaft die folgenden Gruppen genannt:
Methoxy, Ethoxy, Propoxy, Hexoxy, Methoxyethylen, Propoxyethylen, Methoxypropylenyl, Ethoxypropylenyl, Propoxypropylenyl, Oxyethylenylmethylether, Oxyethylenylethylether, Oxyethylenylpropylether, Oxyethylenylhexylether, Bis-(oxyethylenyl-)methylether, Bis-(oxyethylenyl-)ethylether, Bis-(oxyethylenyl-)hexylether, Tetrakis-(oxyethylenyl-)methylether, Tetrakis-(oxyethylenyl-)ethylether.

Die Cyanhydrine II können separat hergestellt, gelagert und nach Bedarf der erfindungsgemäßen Umsetzung zugeführt werden. Es ist aber auch möglich, die Cyanhydrine II unmittelbar vor der erfindungsgemäßen Umsetzung aus den entsprechenden Aldehyden, beispielsweise wie in DE-A 19 50 280 beschrieben, durch Reaktion mit Cyanwasserstoff oder Cyaniden zu erzeugen. Formamid ist als Grundchemikalie im Handel erhältlich, und entsprechendes gilt für die als Katalysatoren zu verwendenden Säuren. Die Ammoniumsalze können beispielsweise durch die Neutralisation wäßriger Lösungen der jeweiligen Säuren mit Ammoniak, Kristallisation und Trocknung erhalten werden.

Überraschenderweise läßt sich mit dem erfindungsgemäßen Verfahren die Abgasmenge, welche bei der Umsetzung entsteht, auf ca. 2 Liter pro Liter Reaktionsmischung verringern. Das im erfindungsgemäßen Verfahren entstehende Abgas enthält vorteilhafterweise keinen Cyanwasserstoff und kann daher problemlos entsorgt werden. Weitere Vorteile des erfindungsgemäßen Verfahrens bestehen darin, daß man mit geringeren Mengen an Katalysatorsäure auskommt und der für eine vollständige Umsetzung erforderliche Formamidüberschuß auf weniger als 2 mol Formamid pro Mol Cyanhydrin II verringert werden kann. Besonders bemerkenswert ist die mit dem erfindungsgemäßen Verfahren erzielbare, beträchtliche Verbesserung der Ausbeute an den entsprechenden α-Formylaminonitrilen I. Beispielsweise erhöht sich die Ausbeute an α-Formylalaninnitril, bezogen auf Acetaldehydcyanhydrin, von 82 auf 95 % der Theorie und bezogen auf Formamid von 75 auf 94 % der Theorie. N-Formylglycinnitril, das nach dem Verfahren der DE-A 19 50 280 bislang nicht zugänglich war, wird erfindungsgemäß in nahezu 90 %iger Ausbeute erhalten.

Die erfindungsgemäß erhältlichen α-Formylaminonitrile können, wie in DE-A 19 50 280 beschrieben, zu den entsprechenden Aminosäuren verseift werden. Des weiteren können aus den erfindungsgemäß erhältlichen α-Formylaminonitrilen durch thermische Cyanwasserstoff-Eliminierung gemäß dem Verfahren der DE-A 16 68 038 und der EP-B 184 074 die entsprechenden N-Formyl-N-Alkene erhalten werden, welche als Monomere Verwendung finden. Beispielsweise kann nach diesem Verfahren das erfindungsgemäß erhältliche α-N-Formylalaninnitril zu N-Vinylformamid pyrolysiert werden, welches gemäß EP-B 71 050 und EP-A 231 901 zu basischen Polymerisaten verarbeitet wird, die beispielsweise als Hilfsmittel bei der Papierveredlung verwendet werden.

### Beispiel 1

Eine Mischung aus 71 g (1 mol) Acetaldehydcyanhydrin (im folgenden als Milchsäurenitril (MSN) bezeichnet, 67,5 g (1,5 mol) Formamid, 6,4 g (0,14 mol) Ameisensäure und 16,4 g (0,26 mol) Ammoniumformiat wurde 6 Stunden unter Rühren auf 90°C erhitzt. Dabei entwichen 220 ml eines Kohlenmonoxid-und Kohlendioxid-haltigen Gasgemisches. Die erhaltene Produktmischung enthielt 92,8 g N-Formylalaninnitril, 6,2 g Ameisensäure, 22,1 g Formamid, 18,9 g Ammoniumformiat und 16,4 g Reaktionswasser. Dieses Produktgemisch wurde unter vermindertem Druck fraktionierend destilliert. α-N-Formylalaninnitril (Siedepunkt: 137°C/2 mbar) wurde in einer Ausbeute von 94,7 %, bezogen auf MSN und in einer Ausbeute von 93,8 % bezogen auf umgesetztes Formamid, erhalten.

### Beispiel 2

Es wurde wie in Beispiel 1 beschrieben gearbeitet, jedoch mit dem Unterschied, daß 8,2 g (0,13 mol) Ammoniumformiat als Cokatalysator zugesetzt wurden. Die Umsetzung wurde wieder bei 90°C durchgeführt. Die Reaktionszeit bis zur vollständigen Umsetzung betrug 11 Stunden.
Ausbeute an α-N-Formylalaninnitril (bez. auf MSN): 94,3 %.

### Vergleichsbeispiel

Es wurde wie in Beispiel 1 beschrieben gearbeitet, jedoch mit dem Unterschied, daß kein Ammoniumformiat zugesetzt wurde. Die Umsetzung wurde wieder bei 90°C durchgeführt. Die Reaktionszeit bis zur vollständigen Umsetzung betrug 18 Stunden.

Ausbeute an α-N-Formylalaninnitril (bez. auf MSN): 87,2 %.

Ausbeute an α-N-Formylalaninnitril (bez. auf Formamid): 75 %.

### Beispiel 3

Es wurde wie in Beispiel 1 beschrieben gearbeitet, jedoch mit dem Unterschied, daß anstelle von Ameisensäure 9,0 g (0,15 mol) Essigsäure und anstelle von Ammoniumformiat 20,0 g (0,26 mol) Ammoniumacetat der Reaktionsmischung zugesetzt wurden. Nach 5-stündiger Reaktion bei 90°C betrug der Umsatz 98,3 %. Das erhaltene Produktgemisch wurde wie in Beispiel 1 beschrieben aufgearbeitet.

Ausbeute an α-N-Formylalaninnitril (bez. auf MSN): 91,8 %.

### Beispiel 4

Es wurde wie in Beispiel 1 beschrieben gearbeitet, jedoch mit dem Unterschied, daß 32,8 g (0,52 mol) Ammoniumformiat als Cokatalysator zugesetzt werden. Nach 3 Stunden Reaktionszeit bei 90°C war die Umsetzung vollständig. Ausbeute an α-N-Formylalaninnitril (bez. auf MSN): 95,7 %.

### Beispiel 5

Eine Mischung aus 22,8 g (0,4 mol) Formaldehydcyanhydrin, 36,1 g (0,8 mol) Formamid, 2,8 g (0,06 mol) Ameisensäure und 6,4 g (0,1 mol) Ammoniumformiat wurde 12 Stunden auf 90°C erhitzt. Anschließend wurde das erhaltene Produktgemisch unter vermindertem Druck fraktionierend destilliert.

Ausbeute an N-Formylglycinnitril (Sdp: 126°/1 mbar): 89,7 % (bez. auf Formaldehydcyanhydrin).

### Beispiel 6

Eine Mischung aus 48 g (0,425 mol) Isovaleraldehydcyanhydrin, 38,3 g (0,85 mol) Formamid, 2,90 g (0,06 mol) Ameisensäure und 6,8 g (0,11 mol) Ammoniumformiat wurde 5 Stunden auf 90°C erhitzt. Anschließend wurde das erhaltene Produktgemisch unter vermindertem Druck fraktionierend destilliert.

Ausbeute an α-N-Formylleucinnitril (bez. auf das Cyanhydrin): 89 %.

### Beispiel 7

Eine Mischung aus 50,5 g (0,5 mol) α-Methoxyacetaldehyd-cyanhydrin, 45 g (1 mol) Formamid, 3,5 g (0,076 mol) Ameisensäure und 8,0 g (0,13 mol) Ammoniumformiat wurde 8 Stunden auf 90°C erhitzt. Anschließend wurde das erhaltene Produktgemisch unter vermindertem Druck fraktionierend destilliert.

Ausbeute an α-N-Formyl-β-methoxy-alaninnitril (bez. auf das Cyanhydrin): 84 %.

## Patentansprüche

1. Verfahren zur Herstellung von α-N-Formylaminonitrilen der allgemeinen Formel I 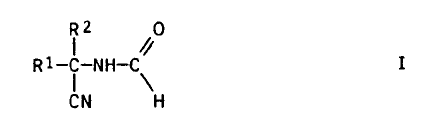 in der die Reste R¹ und R² für Wasserstoff oder für Wasserstoff und unsubstituierte oder mit unter den Reaktionsbedingungen inerten Substituenten substituierte, aliphatische oder heteroaliphatische Reste mit 1 bis 10, cycloaliphatische oder heterocyclooaliphatische Reste mit 3 bis 6, araliphatische Reste mit 7 bis 12, heteroaraliphatische Reste mit 4 bis 12, aromatische Reste mit 6 bis 10 oder heteroaromatische Reste mit 3 bis 10 Kohlenstoffatomen stehen,
durch Umsetzung von Cyanhydrinen der allgemeinen Formel II 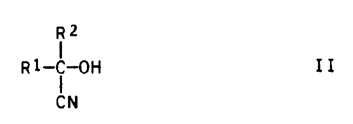 mit Formamid III 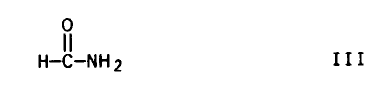 in Gegenwart von Säuren, dadurch gekennzeichnet, daß man der Reaktionsmischung mindestens ein Ammoniumsalz zusetzt.

2. Verfahren nach Anspruch 1, dadurch gekennzeichnet, daß man Cyanhydrine II umsetzt, in denen die Reste R¹ und R² für Wasserstoff oder für Wasserstoff und C₁- bis C₁₀-Alkyl-, C₃- bis C₆-Cycloalkyl-, C₇-bis C₁₁-Aralkyl- oder C₄-C₁₁-Heteroaralkyl-, C₆- bis C₁₀-Aryl-, C₃- bis C₁₀-Heteroaryl- oder für sauerstoffhaltige C₁- bis C₁₀-Heteroalkylgruppen mit 1 bis 5 Sauerstoffatomen stehen.

3. Verfahren nach Anspruch 1, dadurch gekennzeichnet, daß man Cyanhydrine II umsetzt, in denen die Reste R¹ und R² für Wasserstoff oder für Wasserstoff und C₁- bis C₁₀-Alkylgruppen stehen.

4. Verfahren nach Anspruch 1, dadurch gekennzeichnet, daß man die Umsetzung bei 20 bis 150°C durchführt.

5. Verfahren nach Anspruch 1, dadurch gekennzeichnet, daß man die Umsetzung bei Temperaturen von 20 bis 100°C durchführt.

6. Verfahren nach Anspruch 1, dadurch gekennzeichnet, daß man als Ammoniumsalz das Ammoniumsalz derjenigen Säure zusetzt, welche zur Säurekatalyse der Umsetzung benutzt wird.

7. Verfahren nach Anspruch 1, dadurch gekennzeichnet, daß man der Reaktionsmischung 0,05 bis 0,5 Mol Ammoniumformiat pro Mol Cyanhydrin II zusetzt.

8. Verfahren nach Anspruch 1, dadurch gekennzeichnet, daß man die Umsetzung unter dem Eigendruck des Reaktionssystems durchführt.

## Revendications

1. Procédé de préparation d'α-N-formylaminonitriles de formule générale I 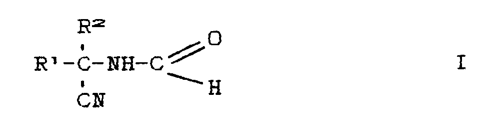 dans laquelle les restes R¹ et R² sont mis pour des atomes d'hydrogène ou pour un atome d'hydrogène et un reste aliphatique ou hétéroaliphatique à 1-10 atomes de carbone, cycloaliphatique ou hétérocycloaliphatique à 3-6 atomes de carbone, araliphatique à 7-12 atomes de carbone, hétéroaraliphatique à 4-12 atomes de carbone, aromatique à 6-10 atomes de carbone ou hétéroaromatique à 3-10 atomes de carbone, non substitué ou substitué par des substituants inertes dans les conditions de la réaction,
par réaction de cyanhydrines de formule générale II 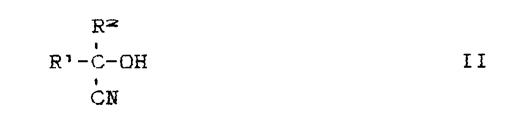 avec du formamide III 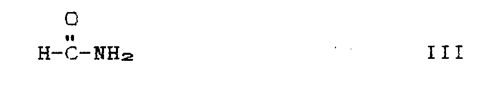 en présence d'acides, caractérisé en ce qu'on ajoute au mélange réactionnel au moins un sel d'ammonium.

2. Procédé selon la revendication 1, caractérisé en ce qu'on met en réaction des cyanhydrines II dans lesquelles les restes R¹ et R² sont mis pour des atomes d'hydrogène ou pour un atome d'hydrogène et un reste alkyle en C₁ à C₁₀, cycloalkyle en C₃ à C₆, aralkyle en C₇ à C₁₁ ou hétéroaralkyle en C₄ à C₁₁, aryle en C₆ à C₁₀, hétéroaryle en C₃ à C₁₀ ou pour des groupements hétéroalkyle en C₁ à C₁₀ oxygénés contenant 1 à 5 atomes d'oxygène.

3. Procédé selon la revendication 1, caractérisé en ce qu'on met en réaction des cyanhydrines II dans lesquelles les restes R¹ et R² sont mis pour des atomes d'hydrogène ou pour un atome d'hydrogène et un groupement alkyle en C₁ à C₁₀.

4. Procédé selon la revendication 1, caractérisé en ce qu'on conduit la réaction à une température de 20 à 150°C.

5. Procédé selon la revendication 1, caractérisé en ce qu'on conduit la réaction à une température de 20 à 100°C.

6. Procédé selon la revendication 1, caractérisé en ce qu'on ajoute, en tant que sel d'ammonium, le sel d'ammonium de l'acide qui est utilisé pour la catalyse acide de la réaction.

7. Procédé selon la revendication 1, caractérisé en ce qu'on ajoute au mélange réactionnel de 0,05 à 0,5 mole de formiate d'ammonium par mole de cyanhydrine II.

8. Procédé selon la revendication 1, caractérisé en ce qu'on conduit la réaction sous la pression propre du système réactionnel.

## Claims

1. A process for preparing an α-formylamino nitrile of the formula I 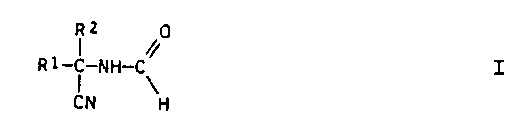 where R¹ and R² are hydrogen or are hydrogen and unsubstituted or substituted, with substituents which are inert under the reaction conditions, aliphatic or heteroaliphatic radicals with 1 to 10, cycloaliphatic or heterocycloaliphatic radicals with 3 to 6, araliphatic radicals with 7 to 12, heteroaraliphatic radicals with 4 to 12, aromatic radicals with 6 to 10 or heteroaromatic radicals with 3 to 10 carbon atoms, by reacting a cyanohydrin of the formula II 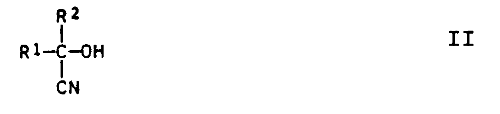 with formamide III 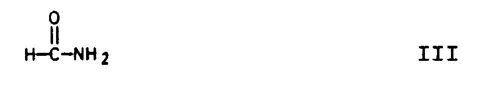 in the presence of acids, which comprises adding at least one ammonium salt to the reaction mixture.

2. A process as claimed in claim 1, wherein R¹ and R² in the cyanohydrin II are hydrogen or are hydrogen and C₁-C₁₀-alkyl, C₃-C₆-cycloalkyl, C₇-C₁₁-aralkyl or C₄-C₁₁-heteroaralkyl, C₆-C₁₀-aryl, C₃-C₁₀-heteroaryl, or C₁-C₁₀-heteroalkyl with 1 to 5 oxygen atoms.

3. A process as claimed in claim 1, wherein R¹ and R² in the cyanohydrin II are hydrogen or are hydrogen and C₁-C₁₀-alkyl.

4. A process as claimed in claim 1, wherein the reaction is carried out at from 20 to 150°C.

5. A process as claimed in claim 1, wherein the reaction is carried out at from 20 to 100°C.

6. A process as claimed in claim 1, wherein the ammonium salt is the salt of that acid used to catalyze the reaction.

7. A process as claimed in claim 1, wherein from 0.05 to 0.5 mole of ammonium formate is added per mole of cyanohydrin II in the reaction mixture.

8. A process as claimed in claim 1, wherein the reaction is carried out under the autogenous pressure of the system.
